# EUROPEAN PATENT APPLICATION

(11) **EP 2 884 767 A1**
(43) Date of publication of application: **17.06.2015**
(21) Application number: 13197428.9
(22) Date of filing: 16.12.2013
(51) Int. Cl.: H04R 25/00, A61B 17/58

(54) **Device for installing an implant for a bone anchored hearing aid**

(71) Applicant: Oticon Medical A/S, 2765 Smørum (DK)
(72) Inventor: Jinton, Lars, SE-43144 Mölndal (SE); Johansson, Martin, DK-2765 Smorum (DK)
(74) Representative: Nielsen, Hans Jørgen Vind

(57) **Abstract**

A method for installing an implant (26) for a bone anchored hearing aid is disclosed. The method comprises the step of providing an incision hole (10) in the soft tissue (2) and a hole (10') in the underlying bone tissue (4) of a subject, which methods comprises the step of anchoring the implant (26) in the hole (10') provided in the bone (4). The method comprises the steps of:
a) punching a cylindrical hole (10) and hereby providing an incision in the soft tissue (2) without initially providing a linear incision;
b) drilling a hole (10') in the underlying bone tissue (4);
c) anchoring the implant (26) in the hole (10').

## Description

### Field of invention

The present invention generally relates to a method and a device for installing an implant. The invention more particularly relates to a method and a device for installing an implant for a bone anchored hearing aid.

### Prior art

Bone anchored hearing aids are applied for the rehabilitation of patients suffering from hearing losses for which traditional hearing aids are insufficient. A typical bone anchored hearing aid consists of an external hearing aid provided with a vibrating transducer connected to a skin-penetrating abutment through a coupling. The abutment may have an interconnection to a screw-shaped fixture anchored in the skull bone. The fixture may be made of titanium and may be provided with a flange to prevent the fixture from being pushed through the skull bone when exposed to a sudden accidental impact.

The abutment penetrates the skin and the subcutaneous tissue in order to establish a direct coupling (direct bone conduction) from a hearing aid processor to the skull bone.

The methods for installing bone anchored hearing aid implant systems are moving toward minimally invasive methods that can be performed quickly in order to minimize intra-operative problems and to achieve a predictable outcome.

The prior art incision techniques are rather complicated and requires that a flap area is provided by making an incision. Typically a scalpel is used to make the incision down to the periosteum along a marking of the incision area and to separate the tissue from the underlying periosteum. Further, all subcutaneous tissue in the graft area is separated from the periosteum. Besides the subcutaneous tissue needs to be carefully separated from the skin graft, and all hair follicles needs to be removed. Furthermore, manual skin thinning typically is required to be performed.

Some attempts have been made to avoid the linear incision techniques to install implants for bone anchored hearing aids. Some of these attempts include punch techniques. The techniques apply a standard biopsy punch that is used to provide a circular incision of 5-12 mm.

These techniques are associated with a number of drawbacks. The drill interfaces the soft tissue and hereby introduces the risk of damaging the tissue due to friction, heat and tearing caused by the action of the drill.

These punching techniques apply punching holes larger than 5 mm in order to allow for introducing irrigation fluid (to cool the bone tissue) during the drilling process. These large punch diameters are not optimal for the soft tissue abutment interface. A large circular incision will prolong the healing time and introduce the risk of granulation tissue formation and subsequent infection. Moreover, the skin thickness needs to be determined pre and/or intra operatively.

Thus there is need for a method that reduces or even eliminates these drawbacks of the prior art.

It is an object of the present invention to provide a standard procedure facilitating a more predictable outcome compared to the incision techniques used today. It is an object to provide a less invasive method that requires a shorter healing time than the prior art methods. It is also an object to provide a method that is fast, uncomplicated as well as patient and economy friendly.

### Summary of the invention

The object of the present invention can be achieved by a method as defined in claim 1 and by a drill guide as defined in claim 12. Preferred embodiments are defined in the dependent sub claims and explained in the following description and illustrated in the accompanying drawings.

The method according to the invention is a method for installing an implant for a bone anchored hearing aid, which method comprises the step of
- providing an incision hole (10) in a soft tissue (2),
- providing a blind hole (10') in the underlying bone tissue (4) of a subject,
- anchoring the implant (26) in the blind hole (10') provided in the bone (4), wherein the method comprises the further steps of:
   a) punching a cylindrical hole (10) and hereby providing a circular incision in the soft tissue (2) by pressuring a sharp blade of a cylindrical hollow punch member (6) through the soft tissue (2);
   b) pulling the cylindrical hollow punch member (6) out of the hole (10) and removing the punched out soft tissue from the hole (10);
   c) inserting a drill member (6) in the hole (10);
   d) drilling a blind hole (10') with the inserted drill member (6) in the underlying bone tissue (4);
   e) anchoring the implant (26) in the blind hole (10').

Hereby it is possible to provide a standard procedure with predictable outcome for installing an implant for a bone anchored hearing aid.

By applying the method according to the invention it is possible to provide a less invasive method that requires shorter healing time compared to the prior art methods.

Moreover, the method according to the invention is faster, more uncomplicated and more patient and economy friendly than the prior art methods.

The method comprises the step of providing an incision hole in the soft tissue and a hole in the underlying bone tissue of a subject.

The soft tissue includes the skin (including the epidermis and the dermis) and the subcutaneous fat. The blind hole in the underlying bone tissue has a width and depth matching the dimensions of the implant so that the implant can be screwed into the hole.

The method comprises the step of anchoring the implant in the blind hole provided in the bone. This anchoring of the implant may be established by screwing the implant into the bone tissue.

The method comprises the step of punching a cylindrical hole and hereby providing a circular incision in the soft tissue without initially providing a linear incision.

Hereby it is possible to reduce the incision area significantly. A prior art incision has a rather large area and the skin graft needs to be sutured. By using the method according to the invention no skin graft needs to be sutured and thus the risk of complications during the healing process is reduced significantly.

When the punching of the cylindrical hole has been carried out the hole in the bone tissue is provided through a drilling process. Drilling the hole in the underlying bone tissue may be carried out by means of any suitable drilling device.

When the cylindrical hole has been provided in the bone structure, the next step of the method is to anchor the implant in the hole.

The anchoring of the implant in the hole in the bone tissue may be carried out by any suitable means. However, in order to provide a reproducible method step it may be an advantage that the implant installation is carried out by means of an electrical drilling equipment. It may be beneficial that low speed setting in the range of 10-20 revolutions per minute (RPM) such as 15 rpm. It may be an advantage that the torque setting is adjusted to suit the quality of the bone tissue. The quality of the bone tissue may be judged by the surgeon during drilling.

The punched soft tissue may be picked out with tweezers or another suitable tool.

It may be an advantage that the area of the circular incision is equal to or smaller than the cross sectional area of the implant.

Hereby it is achieved that the size of the wound can be minimised and thus that optimum healing conditions can be achieved.

It may be an advantage that the area of the circular incision is equal to or smaller than the cross sectional area of the abutment of the implant.

It may be beneficial that the area of the cylindrical hole extends through the soft tissue.

Hereby the hole in the underlying bone tissue can be provided by means of drilling directly into the bone tissue without damaging the soft tissue during the drilling procedure.

It may be an advantage that the method comprises the following steps:
a) inserting a drill guide having a centrally arranged canal and a predefined length in the hole;
b) inserting a drill member into the canal of the drill guide;
c) drilling a blind hole in the bone tissue by means of the drill member.

Hereby it is achieved that the drill guide may stop or reduce the bleeding after punching. The drill guide can protect the soft tissue during drilling, ensure that the correct drill depth is achieved (and thus preventing too deep drilling) and facilitate adequate irrigation. Furthermore, the drill guide makes it possible to ensure that the drilling is carried out perpendicular to the surface of the bone.

Accordingly, the method according to the invention provides a safe and reproducible way of providing a hole in the soft tissue and in the bone tissue and thus to install a bone anchored hearing aid into bone tissue.

It may be advantageous that the method comprises the steps of applying a drilling device having a drill member and a stop member provided at a predefined distance from the distal end of the drill member.

Hereby the depth of the hole provided in the bone tissue by means of the bore member can be controlled. The use of a drill member with a stop member is a major advantage and ensures that the drilling process can be controlled and reproduced. Previously known stop members have the shape of flanges extending away from the drill member and adapted to abut the bone tissue directly when a predefined depth has been reached, but by providing a flange which abuts a drill guide, the flange need not enter the hole in the soft tissue, and further, friction heat generated prior to termination of the drilling due to pressure between the flange or stop member will have no effect on tissue, when the stop member abuts the upper flange of the drill guide.

It may be beneficial that the method comprises the step of verifying that the geometry and angle of the drilled hole is acceptable. The verification may be carried out by using visual inspection and any suitable measurement tools.

It may be an advantage that the hole in the underlying bone tissue is made through drilling carried out perpendicular to the surface of the bone.

This can be ensured by using a drill guide inserted into the punched hole in the soft tissue.

It may be advantageous that the method comprises the step of inserting an expanding drill guide into the drill guide, where the expanding drill guide is configured to radially outwardly push against the inside of the drill guide.

Hereby the wall member of the drill guide and the soft tissue is pushed in place.

It may be beneficial that the method comprises the step of inserting an inner drill guide is inserted into the drill guide, where the inner drill guide is slightly narrower than the inside diameter of the canal in the (outer) drill guide.

An expanding drill guide or an inner drill guide may be used if an initial drilling process is desired. It may be an advantage to provide an initial hole having a smaller diameter before providing the hole into which the implant has to be installed. An initial hole can be used to test if the bone quality is sufficiently good to achieve a firm attachment of the implant. If the bone quality is sufficiently good a hole of greater size may be provide by means of the outer drill guide. Accordingly, the expanding drill guide or the inner drill guide may be removed from the outer drill guide.

It may be advantageous that the method comprises the step of cooling down the hole during drilling the hole by supplying a cooling fluid such as sodium chloride dissolved in water to the bone tissue.

Hereby the bone tissue can be protected against overheating.

It may be beneficial that the method comprises the step of accomplishing a plurality of sequences each comprising a drilling process followed by a drill removal process, where the drill member is removed from the hole in the bone followed by a fluid supplementation process, where a cooling fluid is supplied to the hole in the bone.

Hereby the method ensures that the bone tissue can be sufficiently cooled down to and also bone fragments may be flushed away from the hole.

It may be an advantage that the method comprises the step of removing pieces of tissue from the drilling process, when the drill member is removed from the hole.

When the drill member is removed from the hole, pieces of tissue from the drilling process can be removed e.g. by means of a syringe.

It may be an advantage that the method comprises the step of attaching a healing cap to the implant. It may be an advantage that the healing cap is attached uppermost portion of the abutment just below the top portion.

The healing cap may be made in a semi soft material having a Shore A hardness of 20-75, such as 40-60. The healing cap may be shaped as a circular disk with a central circular aperture. The healing cap may be flexible so that the aperture can be enlarged during attachment to the abutment.

It may be beneficial that the method comprises the step of providing a wrap member between the skin and the healing cap in order to provide a slight pressure to the wound.

Hereby, optimum conditions for healing can be provided. It may be beneficial that the wrap member has a surface structure allowing air to have access to the area below the wrap member. This will facilitate the healing process.

The objects of the invention can be achieved by a drill guide for installing an implant for a bone anchored hearing aid in a hole in a bone under a soft tissue, wherein the drill guide comprises a cylindrical portion provided with a cylindrical canal extending along the longitudinal axis of the cylindrical portion of the drill guide, where the drill guide moreover comprises a flange extending perpendicular to the longitudinal axis of the cylindrical portion of the drill guide, where the drill guide has a length that is greater than the thickness of the soft tissue and where the cylindrical canal is configured to receive a drill member for drilling a hole in the bone.

The use of such drill guide makes it possible to stop the bleeding after punching. The drill guide also protects the soft tissue during a drilling process, where a drill member is inserted into the surgical devise. Besides, the drill guide ensures that the correct drill depth is achieved (and thus preventing too deep drilling). Moreover, the drill guide makes it possible to provide adequate irrigation of the bone tissue during a drilling procedure. Furthermore, the drill guide makes it possible to ensure that the drilling is carried out perpendicular to the surface of the bone.

It may be an advantage that the length of the drill guide is at least three times the outer diameter of the drill guide.

Such geometry is advantageous because it fits the structural dimensions of the soft tissue, the implant and drill member of relevance during installation of an implant for a bone anchored hearing aid.

The objects of the invention may be achieved by a kit for installing an implant for a bone anchored hearing aid in a bone under a soft tissue, wherein the kit comprises a drill guide according to claim 12 and a drilling device comprising a drilling member and a stop member attached to the drilling member at a distance from the distal end of the drilling member, where the distance is greater than the length, where the depth of the hole being drilled by means of the drill member is defined by the difference between the distance and the length.

The stop member ensures that a correct drilling depth can be achieved.

It may be advantageous that the kit comprises a drill guide according to claim 12, where the length of the drill guide is 15-30 mm such as 20 mm, where the inner diameter of the canal is 2.0-4.0 mm such as 3.8 mm and where the outer diameter of the cylindrical portion is 2.5-5.5 mm such as 5.0 mm, where the kit comprises a drill member having a diameter of 2.5-5 mm, such as 3.8 mm, where the kit comprises a punch member having an outer diameter of 3-5 mm such as 4.0 mm, where the kit comprises a implant having a threaded portion having an outer diameter of 2.5-5.5 mm such as 4 mm.

### Description of the Drawings

The invention will become more fully understood from the detailed description given herein below. The accompanying drawings are given by way of illustration only, and thus, they are not limitative of the present invention. In the accompanying drawings:

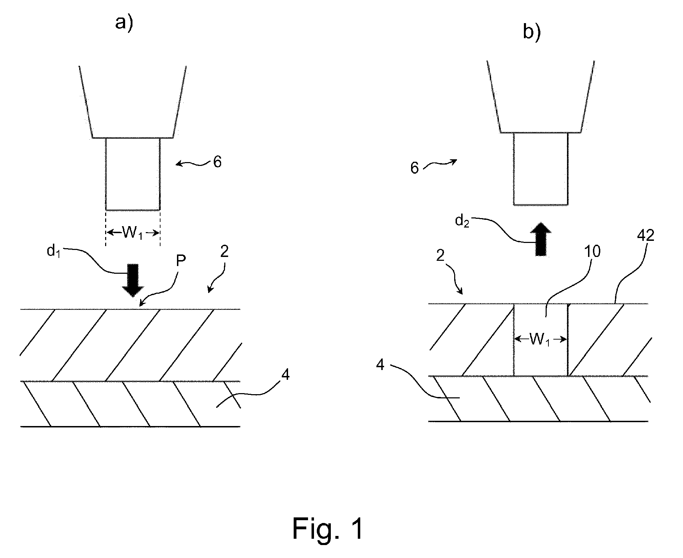

- Fig. 1 a): shows a schematic view of a punch member being used to provide a circular incision;
- Fig. 1 b): shows a schematic view of the punch member being removed from the soft tissue after having provided a circular incision;
- Fig. 2 a): shows a schematic cross-sectional view of a drill guide arranged in a circular incision hole provided by a technique corresponding to the one shown in Fig. 1;

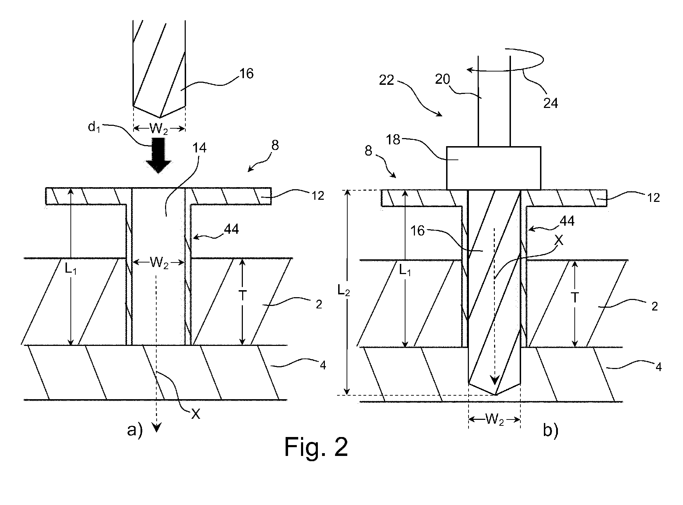

- Fig. 2 b): shows a schematic cross-sectional view of a drill member arranged in the drill guide shown in Fig. 2 a);

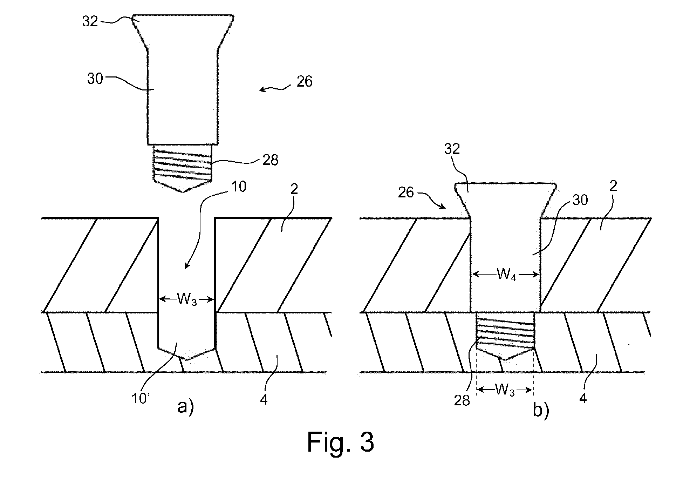

- Fig. 3 a): shows a schematic view of an implant screw arranged above a cylindrical hole provided in the soft tissue and then underlying bone;
- Fig. 3 b): shows a schematic cross-sectional view of the implant screw attached to the bone;

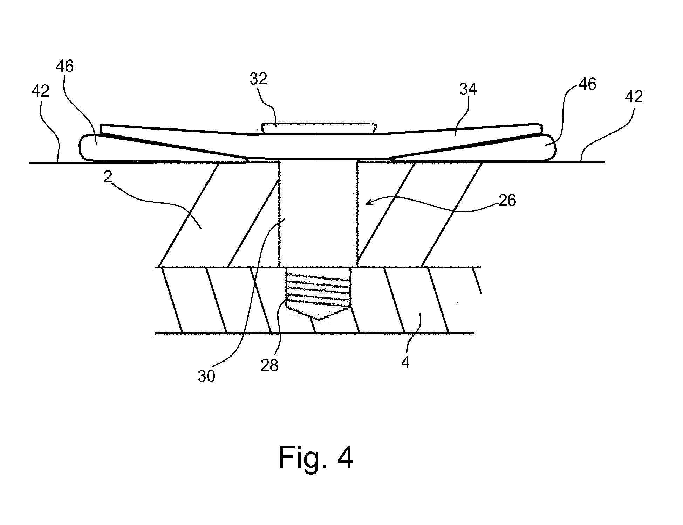

- Fig. 4: shows a schematic view of the implant screw provided with a healing cap;

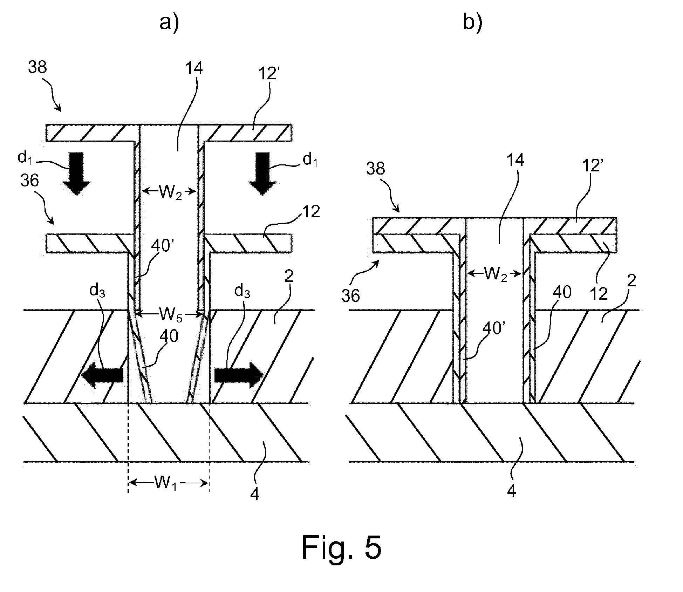

- Fig. 5 a): shows a schematic cross-sectional view of a first drill guide arranged in a circular incision hole and a second drill guide being inserted into the interior of the first drill guide;
- Fig. 5 b): shows a schematic cross-sectional view of the second drill guide being fully inserted into the interior of the first drill guide shown in Fig. 5 a);

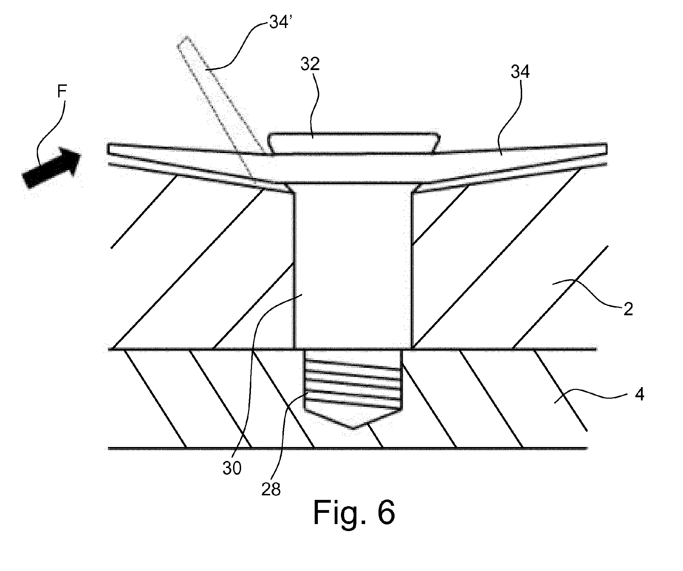

- Fig. 6: shows a schematic view of a healing cap being partly exposed from the soft tissue and

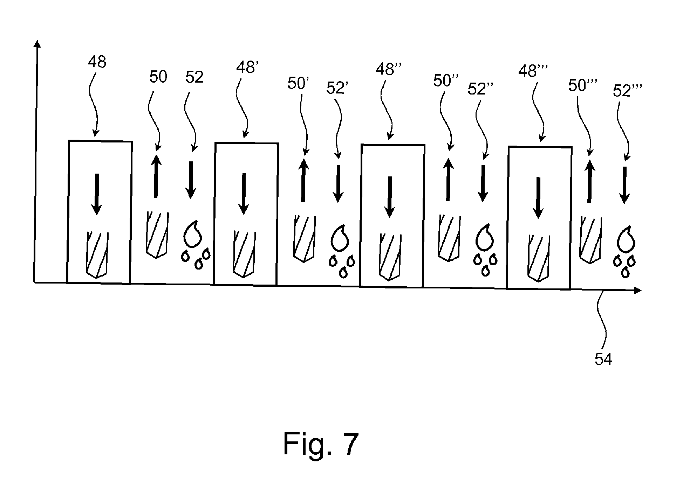

- Fig. 7: shows a schematic view of a plurality of sequences carried out when applying the method according to the invention.

### Detailed description of the invention

Referring now in detail to the drawings for the purpose of illustrating preferred embodiments of the present invention, different steps of the method according to the invention are illustrated in Fig. 1.

Fig. 1 a) shows a schematic view of a punch member 6 being moved towards the soft tissue 2 of a subject. The punch member 6 is moved in the indicated direction d₁ towards the soft tissue 2 of a subject.

The punch member 6 may be a standard punch 6 that is supplied sterile. The punch member 6 may be a single use standard punch.

The punch member 6 has a diameter of 4 mm indicated as the width W₁ at Fig. 1 a). However, the punch member may have another diameter such as 3.0 mm by way of example. Prior to the punching procedure a suitable position P is chosen and the skin is shaved. Preferably, the skin thickness is measured at the chosen position P e.g. by means of a needle and a ruler, where the needle is inserted into the soft tissue. Hereby it is possible to choose an implant having the most suitable abutment length. The method according to the invention may include the step of measuring the bone thickness with ultra sound to ensure that the thickness of the bone tissue 4 is sufficient.

Fig. 1 b) shows a schematic view of the punch member 6 being removed from the soft tissue 2 after having provided a circular incision hole 10 in the soft tissue 2. The soft tissue 2 includes the skin 42 (including the epidermis and the dermis) and the subcutaneous fat.

The punched soft tissue may be picked out with tweezers or another suitable tool (not shown).

After the first punching procedure another punch may be made in order to ensure that the punch member 6 hits the bone 4 and penetrates the periosteum of the bone 4. Tweezers or another suitable tool may be used to pick out the remaining tissue from the hole 19.

Hereafter the periosteum may be scraped off with a raspatorium or another suitable tool. The shape of the skin 42 surrounding the punched hole 10 may be intact in a time period such as 5 minutes after the punching procedure. Thus, the inner diameter or width W₁ of the punched hole 10 will basically correspond to the width W₁ of the punch member 6. Accordingly, the punched hole 10 may have a diameter and width W₁ of about 4 mm.

Fig. 2 a) shows a schematic view of one of the steps of the method according to the invention. Fig. 2 a) shows a cross-sectional view of a drill guide 8 arranged in a circular incision hole provided by a technique corresponding to the one shown in Fig. 1 b).

The drill guide 8 comprises a cylindrical portion 44 provided with a canal 14 extending along the longitudinal axis X of the cylindrical portion 44 of the drill guide 8. The drill guide 8 also comprises a flange 12 extending perpendicular to the longitudinal axis X of the cylindrical portion 44 of the drill guide 8. The length L₁ of the drill guide 8 is greater than the thickness T of the soft tissue 4.

When the drill guide 8 is positioned in the circular incision hole, sodium chloride (NaCl) dissolved in water is introduced into the cylindrical portion 44 of the drill guide 8 e.g. by means of a syringe. Hereby it is achieved that the sodium chloride containing solution is present in the bottom portion of the drill guide 8.

Now the drill guide 8 is ready to receive the drill member 16 that is arranged above the drill guide 8. The drill member 16 needs to be moved in the indicated direction d₁. The drill member 16 may have a diameter or width W₂ of e.g. 3.8 mm.

Fig. 2 b) shows a schematic view of the next step of the method according to the invention. Fig. 2 b) shows a cross-sectional view of a drill device 22 arranged in the drill guide shown in Fig. 2 a).

The drill device 22 comprises a drill member 16 extending along the longitudinal axis X of the cylindrical portion 44 of the drill guide 8. The drill device 22 also comprises a stop member 18 and a rod member 20 extending along longitudinal axis X of the cylindrical portion 44 of the drill guide 8. The stop member 18 is arranged between the rod member 20 and the drill member 16. The stop member 16 bears against the flange 12 of the drill guide 8.

Since the length L₂ of the bore member 16 exceeds the length L₁ of the drill guide 8, the bore member 16 will provide a blind hole 10' in the bone 4. The blind hole 10' is illustrated in Fig. 3 a).

The drilling device 22 is rotated in the clockwise direction 24 and thus a blind hole 10' is provided in the bone 4. The diameter or width W₂ of the drill member 16 that corresponds to the inner diameter of the drill guide 8 may be 3.5-3.95 mm such as 3.8 mm by way of example.

The water in the cylindrical portion 44 of the drill guide 8 will ensure that the bone 4 is cooled down during the drilling procedure.

The drill member 16 may be operated at an operational speed of 1000-3000 RPM e.g. 2000 RPM. The drill member 16 may be pulled up once or more to ensure that the bone is cooled down. Hereafter, the drill member 16 is inserted into the bore again and the drilling procedure is continued. This procedure is explained in further detail with reference to Fig. 7.

It may be an advantage that a cooling fluid (e.g. NaCl dissolved in water) is added to the bone tissue 4 via the drill guide 8 every time the drill member 16 is pulled up.

The drill guide 8 stops the bleeding caused by the punching. Moreover, the drill guide 8 protects the soft tissue 2 during the drilling procedure. Further, the length of the drill guide 8 ensures that the drilling depth is correct. The drill guide 8 hereby prevents drilling too deep into the bone tissue 4.

The drill guide 8 may be a single use invasive device e.g. made in a plastic material and produced by an injection moulding process. The drill guide 8 should be sterile when applied.

The drilling device 22 may be a spiral cylindrical drill having a fixed stop member provided at a predefined position at the drill member 16 in order to provide a blind hole having a predefined depth.

When the drill guide 8 is pulled up from the hole 10 pieces of tissue from the drilling process (soft tissue and bone tissue) and the cooling fluid are sucked up by means of a syringe or another suitable means.

In one preferred embodiment of the method according to the invention the method comprises the step of alternately drilling a short time period e.g. 1-10 seconds, such as 2-5 seconds and pulling the drill member 16 up in order to cool down the drill member 16. This procedure is repeated several times e.g. 2-10 times such as 3-6 times. This is explained further with reference to Fig. 7.

Fig. 3 a) shows a schematic view of an implant screw 26 arranged above a cylindrical hole 10 provided in the soft tissue 2 and a further blind hole 10' provided in the underlying bone 4 of a subject. The blind hole 10' extends as an extension of the hole 10. Both holes 10, 10' have been cleaned by sucking up tissue pieces including soft tissue and bone tissue released during the drilling procedure.

The inner width W₃ of the hole 10 basically corresponds to the diameter of the threaded portion 28 of the implant screw 26. The implant screw 26 comprises a top portion 32, an abutment 30 and a threaded portion 28.

Fig. 3 b) shows a schematic view of the implant screw 26 inserted into the holes 10 in the soft tissue 2 and in the blind hole 10' in the underlying bone 4.

The implant 26 is inserted into the hole 10 and a torque of sufficient magnitude (e.g. 0.1-10 Nm, such as 0.5 Nm) is applied to fix the implant 26 into the bone 4. The implant 26 may be installed with a relative low rotational speed within the range of 1-100, such as 10-30 RPM by way of example.

Fig. 4 shows a schematic view of the implant screw 26 provided with a healing cap 34. The method according to the invention may comprise the step of providing a healing cap 34 to the implant screw 26 after the implant screw has been inserted into the bone 4.

The healing cap 34 is attached to the uppermost portion of the abutment 30 just below the top portion 32. The healing cap 34 may be made in a semi soft material having a Shore A hardness of 20-75, such as 40-60. The healing cap 34 may be shaped as a circular disk with a central circular aperture. The healing cap 34 may be flexible so that the aperture can be enlarged during attachment to the abutment 30.

A wrap member 46 may be provided between the skin 42 and the healing cap 34 in order to provide a slight pressure to the wound for a predefined time period e.g. 1-2 weeks, such as ten days. The wrap member 46 may have a surface structure allowing air to have access to the area below the wrap member 46.

Fig. 5 a) shows a schematic cross-sectional view of a first drill guide 36 arranged in a circular incision hole while a second drill guide 38 having the function of a spacer being inserted into the interior of the first drill guide 36.

The lowermost wall member 40 of the first drill guide 36 is bended radially inward; however, when the second drill guide 38 is moved downward as indicated with the direction d₁ towards the bone 4, these walls 40 are pushed back radially outwardly ase illustrated in Fig. 5 b. The arrows indicate the radial directions d₃.

The inner diameter and width W₅ of the first drill guide 36 is slightly larger than the inner diameter and width W₂ of the second drill guide 38.

The second drill guide 38 is inserted into the first drill guide 36 in order to provide a narrow canal 14 to guide a first bore member of a smaller diameter than the drill member 16 shown in Fig. 2 b).

The width W₂ of the second drill guide 38 may also correspond to the width W₂ of the drill member 16 shown in Fig. 2 b). The function of the second drill guide 38 may be to expand the lowermost wall member 40 of the first drill guide 36 if the wall member 40 has been radially inwardly displaced by the surrounding tissue 2. Accordingly, the second drill guide 38 may be applied to ensure that the canal 14 corresponds to the drill member 16 that is use to provide a blind hole 10' in the bone 4.

Fig. 5 b) shows a schematic cross-sectional view of the second drill guide 38 being fully inserted into the interior of the first drill guide 36. It can be seen that the lowermost wall member 40 of the first drill guide 36 have been pushed back towards the surrounding soft tissue 2 by the lowermost wall member 40' of the second drill guide 38.

The second drill guide 38 provides a canal 14 to guide a drill member (not shown). This drill member may have the same or a smaller diameter than the drill member 16 shown in Fig. 2b.

Fig. 6 shows a schematic view of a healing cap 34 being partly exposed from the soft tissue 2 surrounding the abutment 30. Fig. 6 shows that a portion 34' of the healing cap 34 is moved upwards when a force F is applied. This healing cap 34 may be moved upwards in order to provide a wrap member 46 between the skin 42 and the healing cap 34. This may be done in order to provide a slight pressure to the wound (created by the punching process) for a predefined time period (e.g. 1-2 weeks, such as ten days). The wrap member 46 may have a surface structure allowing air to have access to the area below the wrap member 46.

Fig. 7 shows a schematic view of a plurality of sequence of the method according to the invention. The sequences are plotted against time 54. Each sequence comprises a drilling process 48, 48', 48", 48"' followed by a drill removal process 50, 50', 50", 50"', where the drill member 16 is removed from the blind hole 10' in the bone 4. The drill removal process 50, 50', 50", 50'" is followed by a fluid supplementation process 52, 52', 52", 52"', where a cooling fluid is supplied to the hole 10' in the bone 4 in order to cool the bone 4.

Fig. 7 shows four sequence of equal length. The first comprises a drilling process 48 followed by a drill removal process 50 that is followed by a fluid supplementation process 52. The first sequence is followed by a second sequence comprises a drilling process 48' followed by a drill removal process 50' followed by a fluid supplementation process 52'. The second sequence is followed by a third sequence comprises a drilling process 48" followed by a drill removal process 50" that is followed by a fluid supplementation process 52". The third sequence is followed by a fourth sequence comprises a drilling process 48"' followed by a drill removal process 50"' followed by a fluid supplementation process 52"'.

The method according to the invention may comprise less than four sequences or more than four sequences. Moreover, the duration of the sequences may vary.

During the drilling process 48, 48', 48", 48"' bone tissue is removed by the drill member. By breaking the drilling process up into several sequences the temperature can be kept down. The drill removal process 50, 50', 50", 50'" facilitates removal pieces of tissue from the drilling process. Finally, the fluid supplementation process 52, 52', 52", 52'" makes it possible to provide a new fluid supplementation between the drilling process 48, 48', 48", 48"'.

### List of reference numerals

- 2: - Tissue
- 4: - Bone
- 6: - Punch member
- 8, 36, 38: - Drill guide
- 10: - Hole
- 10': - Blind hole
- 12, 12': - Flange
- 14: - Canal
- 16: - Drill member
- 18: - Stop member
- 20: - Rod member
- 22: - Drilling device
- 24: - Rotational direction
- 26: - Implant
- 28: - Threaded portion
- 30: - Abutment
- 32: - Top portion
- 34: - Healing cap
- 40, 40': - Wall member
- 42: - Skin
- 44: - Cylindrical portion
- 46: - Wrap member
- 48, 48', 48'', 48''': - Drilling process
- 50, 50', 50'', 50''': - Drill removal process
- 52, 52', 52'', 52''': - Fluid supplementation process
- 54: - Time
- F: - Force
- W₁, W₂, W₃, W₄, W₅: - Width
- P: - Position
- X: - Longitudinal axis
- L₁, L₂: - Length
- T: - Thickness

## Claims

1. A method for installing an implant (26) for a bone anchored hearing aid, which method comprises the step of
- providing an incision hole (10) in a soft tissue (2),
- providing a hole (10') in the underlying bone tissue (4) of a subject,
- anchoring the implant (26) in the hole (10') provided in the bone (4), **wherein** the method comprises the further steps of:
a) punching a cylindrical hole (10) and hereby providing a circular incision in the soft tissue (2) by pressuring a sharp blade of a cylindrical hollow punch member (6) through the soft tissue (2);
b) pulling the cylindrical hollow punch member (6) out of the hole (10) and removing the punched out soft tissue from the hole (10);
c) inserting a drill member (6) in the hole (10);
d) drilling a blind hole (10') with the inserted drill member (16) in the underlying bone tissue (4);
e) anchoring the implant (26) in the blind hole (10').

2. A method according to claim 1, **wherein** the area of the circular incision is equal to or smaller than the cross sectional area of the implant (26).

3. A method according to claim 1, **wherein** the area of the cylindrical hole (10) extends through the soft tissue (2).

4. A method according to claim 1, **wherein** the method comprises the following steps:
a) inserting a drill guide (8, 36, 38) having a centrally arranged canal (14) and a predefined length (L₁) in the hole (10);
b) inserting a drill member (16) into the canal (14) of the drill guide (8, 36) or expanding drill guide (38);
c) drilling a blind hole (10') in the bone tissue (4) by means of the drill member (16).

5. A method according to claim 4, **wherein** the method comprises the steps of driving the drill member (16) through a drilling device (22) and stop driving the drill member (16) when a stop member (18) provided at a predefined distance from the distal end of the drill member (16) abuts an upper surface of the drill guide (8, 36) or upper surface of expanding drill guide (38).

6. A method according to claim 1, **wherein** the blind hole (10') in the underlying bone tissue (4) is made through drilling carried out perpendicular to an upper surface of the bone (4).

7. A method according to claim 4, **wherein** the method comprises the step of inserting an expanding drill guide (38) into the drill guide (8, 36), where the expanding drill guide (38) is configured to radially outwardly push against the inside of the drill guide (8, 36).

8. A method according to claim 4, **wherein** the method comprises the step of cooling down the hole (10') during drilling the hole (10') by supplying a cooling fluid such as sodium chloride dissolved in water to the bone tissue 4.

9. A method according to claim 8, **wherein** the method comprises the step of accomplishing a plurality of sequences each comprising a drilling process (48, 48', 48", 48"') followed by a drill removal process (50, 50', 50", 50"'), where the drill member (16) is removed from the blind hole (10') in the bone (4), followed by a fluid supplementation process (52, 52', 52", 52"'), where a cooling fluid is supplied to the blind hole (10') in the bone (4).

10. A method according to claim 1, **wherein** the method comprises the step of removing pieces of tissue from the drilling process, when the drill member (16) is removed from the blind hole (10').

11. A method according to claim 10, **wherein** the method comprises the step of providing a wrap member (46) between the skin (42) and the healing cap (34) in order to provide a slight pressure to the wound.

12. A drill guide (8, 36, 38) for installing an implant (26) for a bone anchored hearing aid in a blind hole (10') in a bone (4) under a soft tissue (2), **wherein** the drill guide (8, 36, 38) comprises a cylindrical portion (44) provided with a cylindrical canal (14) extending along the longitudinal axis (X) of the cylindrical portion (44) of the drill guide (8, 36, 38), where the drill guide (8, 36, 38) moreover comprises a flange (12) extending perpendicular to the longitudinal axis (X) of the cylindrical portion (44) of the drill guide (8, 36, 38), where the drill guide (8, 36, 38) has a length (L₁) that is greater than the thickness (T) of the soft tissue (2) and where the cylindrical canal (14) is configured to receive a drill member (16) for drilling a blind hole (10') in the bone (4).

13. A drill guide (8, 36, 38) according to claim 12, **wherein** the length (L₁) of the drill guide (8, 36, 38) is at least three times the outer diameter of the drill guide (8, 36, 38).

14. A drill guide (8) according to claim 12, **wherein** the drill guide (8) is a solitary member, having an outer cylindrical wall which abuts the soft tissue when inserted and an inner cylindrical wall surface which guides a drill (16) during generation of a blind hole (10').

15. A drill guide (36,38) according to claim 14, wherein an outer tissue abutting drill guide (36) is provided, with flexible outer walls, and an inner drill guide (38) is provided with in-flexible walls, whereby the inner drill guide (38) is adapted to expand the outer tissue abutting drill guide (36).

16. A kit for installing an implant (26) for a bone anchored hearing aid in a bone (4) under a soft tissue (2), **wherein** the kit comprise a drill guide (8, 36, 38) according to claim 12 and a drilling device (22) comprising a drilling member (16) and a stop member (18) attached to the drilling member (16) at a distance (L₂) from the distal end of the drilling member (16), where the distance (L₂) is greater than the length (L₁), where the depth of the blind hole (10') drilled by drill member (16) is defined by the difference between the distance (L₂) and the length (L₁).

17. A kit according to claim 16, **wherein** the kit comprises a drill guide (8, 36, 38) according to claim 12, where the length (L₁) of the drill guide (8, 36, 38) is 15-30 mm such as 20 mm, where the inner diameter (W₂) of the canal (14) is 2.0-4.0 mm such as 3.8 mm and where the outer diameter (W₁) of the cylindrical portion (44) is 2.5-5.5 mm such as 5.0 mm, where the kit comprises a drill member (16) having a diameter of 2.5-5 mm, such as 3.8 mm, where the kit comprises a punch member (6) having an outer diameter (W₁) of 3-5 mm such as 4.0 mm, where the kit comprises a implant (26) having a threaded portion (28) having an outer diameter (W₃) of 2.5-5.5 mm such as 4 mm.
